(19) Europäisches Patentamt
European Patent Office
Office européen des brevets

(11) Veröffentlichungsnummer: **0 247 481**
**B1**

(12) # EUROPÄISCHE PATENTSCHRIFT

(45) Veröffentlichungstag der Patentschrift: **08.11.89**

(51) Int. Cl.⁴: **C 07 C 53/50**, C 07 C 51/58 // C07D305/08

(21) Anmeldenummer: **87107160.1**

(22) Anmeldetag: **18.05.87**

(54) **Verfahren zur Herstellung von 2,2-Bis-chlormethylalkan-carbonsäurechloriden.**

(30) Priorität: **30.05.86 DE 3618142**

(43) Veröffentlichungstag der Anmeldung: **02.12.87 Patentblatt 87/49**

(45) Bekanntmachung des Hinweises auf die Patenterteilung: **08.11.89 Patentblatt 89/45**

(84) Benannte Vertragsstaaten: **BE CH DE FR GB IT LI NL**

(56) Entgegenhaltungen:
**EP-A- 0 132 733**
**DE-C- 1 907 117**

**PATENT ABSTRACTS OF JAPAN, unexamined applications, C Field, Band 9, Nr. 293(C-315), 20. November 1985 THE PATENT OFFICE JAPANESE GOVERNMENT Seite 81 C 315**

(73) Patentinhaber: **BAYER AG, D-5090 Leverkusen 1 Bayerwerk (DE)**

(72) Erfinder: **Fiege, Helmut, Dr., Walter-Flex-Strasse 23, D-5090 Leverkusen 1 (DE)**
Erfinder: **Jautelat, Manfred, Dr., Müllersbaum 28, D-5093 Burscheid (DE)**
Erfinder: **Arlt, Dieter, Prof. Dr., Rybniker Strasse 2, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein neues Verfahren zur Herstellung und von teilweise bekannten 2,2-Bis-chlormethyl-alkancarbonsäurechloriden, die als Zwischenprodukte zur Synthese von Stoffen mit fungizider bzw. herbizider Wirksamkeit verwendbar sind.

Es ist bereits bekannt, dass bei der Umsetzung von 3-Alkyl-oxetan-3-carbonsäuren mit Thionylchlorid nur die Carboxylgruppe in eine Chlorcarbonyl-Gruppe umgewandelt wird, der Oxetanring sich jedoch nicht verändert (vgl. DE-PS 1 907 117). Im übrigen sind keine Umsetzungen bekannt, in denen der Oxetan-Ring von Oxetan-3-carbonsäuren mit Thionylchlorid oder anderen anorganischen Säurechloriden reagiert.

Es wurde nun gefunden, dass sich 2,2-Bis-chlormethyl-alkancarbonsäurechloride der Formel

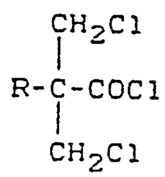

(I)

in welcher
R für Wasserstoff, Alkyl, Cycloalkyl oder gegebenenfalls substituiertes Phenyl steht,
herstellen lassen, indem man Oxetan-3-carbonsäuren der Formel

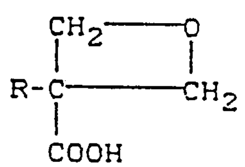

(II)

in welcher
R die oben angegebene Bedeutung hat,
oder deren Salze mit anorganischen Säurechloriden gegebenenfalls in Gegenwart eines Katalysators und gegebenenfalls in Gegenwart eines Verdünnungsmittels bei Temperaturen zwischen 20° C und dem Siedepunkt des Reaktionsgemisches umsetzt.

Der Verlauf des erfindungsgemässen Verfahrens ist als äusserst überraschend zu bezeichnen. So konnte im Hinblick auf den bekannten Stand der Technik nicht davon ausgegangen werden, dass sich Oxetan-3-carbonsäuren der Formel (I) glatt zu 2,2-Bis-chlormethyl-alkancarbonsäurechloriden der Formel (I) umsetzen lassen. Es war nämlich zu erwarten, dass eine derartige Chlorierung erst nach vorheriger hydrolytischer Öffnung des Oxetanringes abläuft.

Das erfindungsgemässe Verfahren zeichnet sich durch eine Reihe von Vorteilen aus. So sind die benötigten Ausgangsstoffe einfach und auch in grösseren Mengen zugänglich. Weiterhin sind auch die erforderlichen Reaktionskomponenten preisgünstig und problemlos zu handhaben. Besonders vorteilhaft ist, dass die gewünschten Produkte in sehr hoher Ausbeute und hervorragender Reinheit anfallen. Im übrigen bereitet die Aufarbeitung der nach der Umsetzung vorliegenden Reaktionsgemische keinerlei Schwierigkeiten.

Verwendet man 3-Methyl-oxetan-3-carbonsäure als Ausgangsstoff, Thionylchlorid als Säurechlorid und Dimethylformamid (= DMF) als Katalysator, so kann der Verlauf des erfindungsgemässen Verfahrens durch das folgende Formelschema veranschaulicht werden:

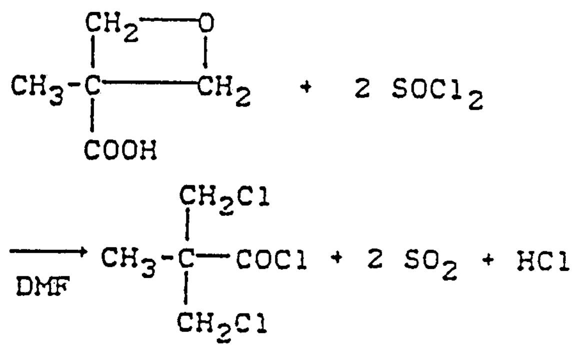


Die bei dem erfindungsgemässen Verfahren als Ausgangsstoffe benötigten Oxetan-3-carbonsäuren sind durch die Formel (II) allgemein definiert. In dieser Formel steht R vorzugsweise für Wasserstoff, Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 6 Kohlenstoffatomen substituiertes Phenyl.

Besonders bevorzugt sind diejenigen Stoffe der Formel (II), in denen R für Wasserstoff, Alkyl mit 1 bis 8 Kohlenstoffatomen, insbesondere mit 1 bis 6 Kohlenstoffatomen, für Cycloalkyl mit 3 bis 6 Kohlenstoffatomen oder für gegebenenfalls durch Fluor, Chlor, Brom und/oder Alkyl mit 1 bis 4 Kohlenstoffatomen substituiertes Phenyl steht.

Vorzugsweise verwendbar sind auch Salze von Oxetan-3-carbonsäuren, wie zum Beispiel deren Alkalimetall- oder Erdalkalimetallsalze. Besonders bevorzugt sind Natrium- oder Kalium-Salze, sowie Magnesium- oder Calcium-Salze.

Als Beispiele für Oxetan-3-carbonsäuren der Formel (II) seien genannt:

Oxetan-3-carbonsäure, 3-Methyl-oxetan-3-carbonsäure, 3-Ethyl-oxetan-3-carbonsäure, 3-Propyl-oxetan-3-carbonsäure, 3-Isopropyloxetan-3-carbonsäure, 3-Butyl-oxetan-3-carbonsäure, 3-Phenyl-oxetan-3-carbonsäure, 3-Cyclohexyl-oxetan-3-carbonsäure und 3-(4-Chlorphenyl)-oxetan-3-carbonsäure.

Die Oxetan-3-carbonsäuren der Formel (II) und deren Salze sind teilweise bekannt. So lassen sich Oxetan-3-carbonsäuren nach einem bekannten Verfahren herstellen, indem man 3-Hydroxymethyl-oxetane in flüssiger Phase in Gegenwart von Kupfer/Chrom/Barium enthaltenden Katalysatoren bei Temperaturen zwischen 190 und 270° C dehydriert. Die dabei entstehenden Oxetan-3-carbonsäureester, die das als Ausgangsmaterial eingesetzte 3-Hydroxymethyl-oxetan als Alkoholkomponente enthalten, werden in einem weiteren Reaktionsschritt zu Oxetan-3-carbonsäuren verseift (vgl. DE-PS 1 907 117).

Die Oxetan-3-carbonsäuren der Formel (II) und deren Salze lassen sich auch nach einem neuen Verfahren herstellen, indem man 3-Hydroxymethyl-oxetane der Formel

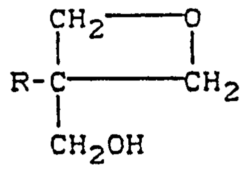

(III)

in welcher

R die oben angegebene Bedeutung hat,

in wässerig-alkalischem Medium mit Sauerstoff oder Sauerstoff enthaltenden Gasen bei Temperaturen zwischen 0° C und dem Siedepunkt des Reaktionsgemisches an einem Palladium- und/oder Platinkatalysator, gegebenenfalls in Gegenwart eines Aktivators umsetzt und danach gegebenenfalls ansäuert.

Verwendet man bei dem obigen Verfahren zur Herstellung von Oxetan-3-carbonsäuren 3-Methyl-3-hydroxymethyl-oxetan als Ausgangsstoff, Sauerstoff als Oxidationsmittel, Palladium und Aktivkohle unter Zusatz von Bismut-nitrat als Katalysator, wässerige Natronlauge als Reaktionsmedium und verdünnte wässerige Schwefelsäure zum Ansäuern, so kann der Verlauf dieses Verfahrens durch das folgende Formelschema veranschaulicht werden:

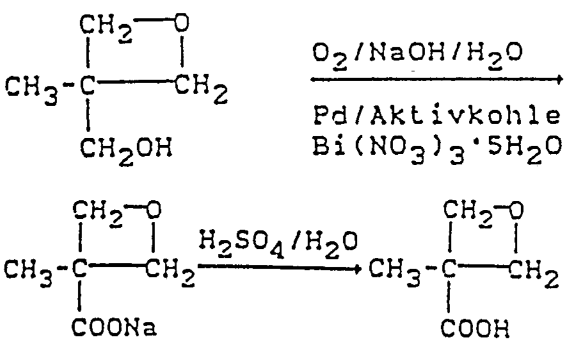


Die bei dem obigen Verfahren zur Herstellung von Oxetan-3-carbonsäuren als Ausgangsstoffe benötigten 3-Hydroxymethyl-oxetane sind durch die Formel (III) allgemein definiert. In dieser Formel hat R vorzugsweise diejenigen Bedeutungen, die bereits im Zusammenhang mit der Beschreibung der Oxetan-3-carbonsäuren der Formel (II) vorzugsweise für diesen Rest genannt wurden.

Als Beispiele für 3-Hydroxymethyloxetane der Formel (III) seien genannt:

3-Methyl-3-hydroxymethyl-oxetan, 3-Ethyl-3-hydroxymethyl-oxetan, 3-Propyl-3-hydroxymethyl-oxetan, 3-Isopropyl-3-hydroxymethyl-oxetan und 3-Butyl-3-hydroxymethyl-oxetan, 3-Hydroxymethyl-oxetan, 3-Phenyl-3-hydroxymethyl-oxetan, 3-(4-Chlorphenyl)-3-hydroxymethyl-oxetan und 3-Cyclohexyl-3-hydroxymethyl-oxetan.

Die 3-Hydroxymethyl-oxetane der Formel (III) sind bekannt oder lassen sich nach bekannten Methoden in einfacher Weise herstellen (vgl. Houben-Weyl, «Methoden der organischen Chemie», 4. Auflage, Bd. VI/3, Seite 493 ff. Georg-Thieme-Verlag, Stuttgart 1965). So können 3-Hydroxymethyl-oxetane der Formel (III) zum Beispiel durch Abspaltung von Kohlendioxid aus den entsprechenden cyclischen Carbonaten erhalten werden.

Bei der Durchführung des obigen Verfahrens zur Herstellung von Oxetan-3-carbonsäuren kommen als Katalysatoren alle üblichen Palladium- und Platin-Katalysatoren sowie deren Gemische in Betracht. Die Katalysatoren können zusätzlich noch mit Aktivatoren oder Gemischen verschiedener Aktivatoren kombiniert werden. Als Aktivatoren kommen hierbei vorzugsweise Blei, Bismut, Bleiverbindungen und Bismutverbindungen sowie deren Gemische in Frage.

Bei der Durchführung des neuen Verfahrens zur Herstellung von Oxetan-3-carbonsäuren der Formel (II) können das als Katalysator zu verwendende Platin oder Palladium oder Gemische, die diese Metalle enthalten, in üblicher Weise eingesetzt werden. So können die Stoffe in elementarer Form, z. B. als sogenanntes Platin- oder Palladium-Mohr, gegebenenfalls in Kombination mit anderen Platinmetallen, oder auch in Form von Verbindungen, wie z. B. den Oxiden, zugegeben werden.

Das Platin oder das Palladium können auch auf einem Träger aufgebracht sein. Als Träger geeignet sind beispielsweise Aktivkohle, Graphit, Kieselgur, Kieselgel, Spinelle, Aluminiumoxid, Asbest, Calciumcarbonat, Bariumsulfat oder auch organische Trägermaterialien.

Bevorzugt werden als Trägermaterial Aktivkohlen, beispielsweise sogenannte Medizinalkohlen oder aus Holz hergestellte Aktivkohlen, wie sie vielfach für Entfärbungszwecke verwendet werden, eingesetzt.

Der Platin- und/oder Palladium-Gehalt der Trägerkatalysatoren kann innerhalb eines grösseren Bereiches variiert werden. Im allgemeinen verwendet man Trägerkatalysatoren, in denen der Gehalt an diesen Metallen zwischen 0,01 und 20 Gew.-%, vorzugsweise zwischen 0,1 und 15 Gew.-% liegt.

Auch die Mengen, in denen die Platin- und/oder Palladium-Katalysatoren eingesetzt werden, können in einem grösseren Bereich variiert werden. Die Mengen hängen unter anderem von der gewünschten Oxidationsgeschwindigkeit ab. Im allgemeinen wählt man die Katalysatormenge so, dass zwischen 0,01 und 20 g, vorzugsweise zwischen 0,05 und 10 g, Platin und/oder Palladium pro Mol an 3-Hydroxymethyl-oxetan der Formel (III) im Reaktionsgemisch anwesend sind.

Bei der Durchführung des obigen Verfahrens ist es auch möglich, Platin in Kombination mit Palladium als Katalysator einzusetzen.

Die Aktivität und/oder Selektivität der Platinkatalysatoren wird bei dem obigen Verfahren durch die Gegenwart von Blei und/oder Bismut und/oder deren Verbindungen als Aktivator erheblich gesteigert.

Palladiumkatalysatoren weisen auch ohne Zusatz der vorgenannten Aktivatoren schon eine so überraschend hohe Aktivität und Selektivität auf,

dass bei ihnen gegebenenfalls auf den Zusatz der vorgenannten Aktivatoren verzichtet werden kann.

Der Zusatz der vorgenannten Aktivatoren wirkt sich auch positiv auf die Wiederverwendbarkeit der Katalysatoren aus.

Die Mengen, in denen diese Aktivatoren bei der Durchführung des obigen Verfahrens gegebenenfalls eingesetzt werden, können in einem grösseren Bereich variiert werden. Die Aktivatorwirkung macht sich bereits bei Zusätzen von $5 \times 10^{-6}$ Mol Metall oder Metallverbindung pro Mol 3-Hydroxymethyl-oxetan bemerkbar. Es können auch 0,1 Mol oder mehr Aktivator pro Mol 3-Hydroxymethyl-oxetan eingesetzt werden, jedoch bieten diese hohen Zusätze im allgemeinen keinen Vorteil. Üblicherweise werden die Aktivatoren in Mengen von etwa $1 \times 10^{-5}$ bis $1 \times 10^{-1}$ Mol, vorzugsweise $2 \times 10^{-5}$ bis $2 \times 10^{-2}$, pro Mol zu oxidierendem 3-Hydroxymethyl-oxetan zugegeben.

Die bei der Durchführung des obigen Verfahrens gegebenenfalls als Aktivatoren zu verwendenden Metalle können in elementarer Form und/oder in Form ihrer Verbindungen, z. B. als Oxide, Hydroxide, Oxidhydrate oder Sauerstoffsäuren oder als Salze von Wasserstoffsäuren, wie Chloride, Bromide, Jodide, Sulfide, Selenide, Telluride, oder als Salze von anorganischen Sauerstoffsäuren, wie Nitrate, Nitrite, Phosphite, Phosphate, Arsenite, Arsenate, Antimonite, Antimonate, Bismutate, Stannate, Plumbate, Selenite, Selenate, Tellurite, Tellurate, Borate oder als Salze von Sauerstoffsäuren, die von Übergangsmetallen abstammen, wie Vanadate, Niobate, Tantalate, Chromate, Molybdate, Wolframate, Permanganate, oder als Salze organischer aliphatischer oder aromatischer Säuren, wie Formiate, Acetate, Propionate, Benzoate, Salicylate, Lactate, Mandelate, Glyoxylate, Oxetan-carboxylate, Citrate, Phenolate, oder als Komplexverbindungen oder als metallorganische Verbindung eingesetzt werden.

Die Aktivatoren können im Reaktionsgemisch jeweils löslich, teilweise löslich oder unlöslich sein.

Es ist auch möglich, die Aktivatoren in Kombination mit anderen nicht als Aktivator beanspruchten Elementen oder Verbindungen in das obige Verfahren einzusetzen.

Die bei der Durchführung des obigen Verfahrens gegebenenfalls einzusetzenden Aktivatoren können in unterschiedlichen und auch in gemischten Wertigkeitsstufen vorliegen. Es können auch Änderungen in der Wertigkeit während der Reaktion eintreten. Sofern die Aktivatoren nicht bereits als Oxide und/oder Hydroxide zugegeben werden, ist es möglich, dass sie sich im alkalischen Medium ganz oder teilweise in diese umwandeln. Nach der Reaktion kann der Platin und/oder Palladiumkatalysator mit dem Aktivator (soweit dieser ungelöst geblieben ist) abfiltriert und für weitere Oxidationsreaktionen verwendet werden. Verluste an Platin- bzw. Palladiumkatalysator und/oder an Aktivator sind gegebenenfalls zu ersetzen.

Der Aktivator kann als Feststoff, vorzugsweise in feinverteilter Form, oder in gelöster Form den Reaktionskomponenten zugesetzt werden. Man kann den Aktivator auch schon bei der Herstellung des Platin- bzw. Palladium-Katalysators zugeben oder den Platin- bzw. Palladiumkatalysator mit dem Aktivator imprägnieren. Der Aktivator kann auch als Trägermaterial für das Platinmetall dienen.

Die Oxidation nach dem obigen Verfahren erfolgt in wässerigalkalischem Medium bei einem pH-Wert $>7$. Die Einstellung des geeigneten pH-Wertes erfolgt durch Zugabe von Alkalien. Als Alkalien kommen Verbindungen der Alkali- und/oder Erdalkalimetalle in Betracht, wie die Hydroxide, Carbonate, Hydrogencarbonate, Phosphate und Borate. Bevorzugt werden die Hydroxide und/oder Carbonate des Natriums und/oder Kaliums als Alkali eingesetzt.

Da bei dem obigen Verfahren pro Mol gebildeter Säure 1 Mol Alkali ($OH^{\ominus}$) verbraucht wird, liegt die einzusetzende Alkalimenge bei etwa 1 Mol Alkali pro Mol 3-Hydroxymethyl-oxetan. Im allgemeinen werden etwa 1 bis 1,5 Mol Alkali pro Mol 3-Hydroxymethyl-oxetan eingesetzt.

Höhere Verhältnisse können angewendet werden, bringen jedoch gewöhnlich keine wesentlichen Vorteile mehr. Wenn es erwünscht ist, dass vom eingesetzten 3-Hydroxymethyl-oxetan nur ein Teil zur Oxetan-3-carbonsäure oxidiert wird, kann auch entsprechend weniger Alkali eingesetzt werden.

Das Alkali kann zu Beginn auf einmal oder auch erst im Verlauf der Reaktion intermittierend oder kontinuierlich dem Reaktionsgemisch hinzugefügt werden.

Die 3-Hydroxymethyl-oxetane werden bevorzugt in wässeriger Lösung oxidiert. Es können aber auch noch andere inerte organische Substanzen, beispielsweise Lösungsmittel, wie tert.-Butanol, Aceton, Dioxan und/oder Toluol anwesend sein. Die 3-Hydroxymethyl-oxetane werden im allgemeinen in Form einer 2 bis 40%igen Lösung eingesetzt. Welche Konzentration zweckmässig ist, hängt unter anderem von der gewünschten Reaktionsgeschwindigkeit ab. Letztere nimmt bei höheren 3-Hydroxymethyl-oxetan-Konzentrationen allmählich ab. Es ist auch möglich, Gemische verschiedener 3-Hydroxymethyl-oxetane zu oxidieren.

Die Reaktionstemperaturen können bei der Durchführung des obigen Oxidations-Verfahrens innerhalb eines grösseren Bereiches variiert werden. So kann die Reaktionstemperatur zwischen dem Erstarrungspunkt und dem Siedepunkt des Reaktionsgemisches liegen. Die im Einzelfall anzuwendende Reaktionstemperatur richtet sich unter anderem nach dem Katalysatorsystem, der Katalysatormenge, der Alkalikonzentration, den Substanzeigenschaften der Edukte und Produkte und den technischen Gegebenheiten, wie zum Beispiel gewünschte Reaktionsgeschwindigkeit oder Wärmeabfuhr. Im allgemeinen arbeitet man bei Temperaturen zwischen 0° C und dem Siedepunkt des Reaktionsgemisches, vorzugsweise zwischen 40° C und 100° C.

Die Reihenfolge, in der der Platin- und/oder

Palladium-Katalysator sowie gegebenenfalls Aktivator, wässeriges Alkali und 3-Hydroxymethyl-oxetan zusammengegeben werden, ist beliebig. So können der Platin- und/oder Palladium-Katalysator sowie gegebenenfalls Aktivator der Mischung oder Lösung aus wässerigem Alkali und 3-Hydroxymethyl-oxetan zugesetzt werden. Man kann auch Platin- und/oder Palladium-Katalysator sowie gegebenenfalls Aktivator vorlegen und die Mischung aus wässerigem Alkali und 3-Hydroxymethyl-oxetan zusetzen. Schliesslich ist es auch möglich, den Platin- und/oder Palladium-Katalysator, einen Teil des wässerigen Alkalis sowie gegebenenfalls Aktivator vorzulegen und 3-Hydroxymethyl-oxetan dann zusammen mit dem restlichen Alkali hinzuzufügen. Ferner ist es möglich, den Aktivator der Mischung der übrigen Komponenten zuzusetzen.

Im allgemeinen wird das obige Oxidations- Verfahren so durchgeführt, dass man Sauerstoff oder Sauerstoff enthaltende Gase, wie Luft, mit dem Reaktionsgemisch, das wässeriges Alkali, den Platin- und/oder Palladium-Katalysator, gegebenenfalls Aktivator sowie 3-Hydroxymethyl-oxetan enthält, in intensiven Kontakt bringt. Der Katalysator braucht im Reaktionsgemisch nicht als Pulver suspendiert vorzuliegen, sondern kann auch in gekörnter Form als Festbett angeordnet sein, das von den übrigen Komponenten durchströmt wird.

Der Druck kann bei der Durchführung des obigen Oxidations-Verfahrens innerhalb eines grösseren Bereiches variiert werden. Im allgemeinen arbeitet man unter Drucken zwischen 0,5 und 10 bar. Vorzugsweise führt man die Oxidation bei Atmosphärendruck durch.

Der Verlauf der Umsetzung kann durch Messung der aufgenommenen Sauerstoffmenge verfolgt werden. Die Umsetzung wird abgebrochen, wenn die für die Herstellung der jeweiligen Oxetan-3-carbonsäure theoretisch erforderliche Sauerstoffmenge aufgenommen worden ist. Im allgemeinen hört die Sauerstoffaufnahme in diesem Stadium von selbst auf, oder sie verlangsamt sich deutlich.

Die Aufarbeitung erfolgt bei der Durchführung des obigen Oxidations-Verfahrens nach üblichen Methoden. Im allgemeinen geht man so vor, dass man den Katalysator sowie gegebenenfalls vorhandenen ungelösten Aktivator zum Beispiel durch Filtration abtrennt. Die erhaltenen Alkalisalzlösungen der Oxetan-3-carbonsäuren können, gegebenenfalls nach vorherigem Eindampfen, als solche weiterverwendet werden. Man kann die Alkalisalzlösungen der Oxetan-3-carbonsäuren auch vollständig, also bis zur Trockne, eindampfen und den verbleibenden Salzrückstand weiterverwenden. Wenn die freien Oxetan-3-carbonsäuren hergestellt werden sollen, geht man im allgemeinen so vor, dass man das verbleibende Reaktionsgemisch gegebenenfalls nach vorherigem Einengen unter vermindertem Druck mit verdünnter Mineralsäure ansäuert, dann mit einem in Wasser wenig löslichen organischen Solvens extrahiert und die organische Phase, gegebenenfalls nach vorherigem Trocknen, einengt. Als Mineralsäuren können hierbei vorzugsweise Salzsäure, Schwefelsäure oder Phosphorsäure eingesetzt werden. Als organische Solventien für die Extraktion kommen vorzugsweise Ether, wie Diethylether und Diisopropylether, weiterhin Ketone, wie Methylisobutylketon, und ausserdem gegebenenfalls halogenierte aliphatische oder aromatische Kohlenwasserstoffe, wie Methylenchlorid, Chloroform oder Toluol, in Betracht. Es ist auch möglich, die jeweilige Oxetan-3-carbonsäure aus den zunächst anfallenden wässerigen Alkalisalz-Lösungen an einem Kationenaustauscher freizusetzen und durch schonendes Eindampfen der wässerigen Lösung zu isolieren. Bei unvollständigem Umsatz des verwendeten 3-Hydroxymethyloxetans kann man dieses vor dem Ansäuern durch Extraktion der wässerigen Alkalisalz-Lösung mit einem in Wasser wenig löslichen organischen Solvens entfernen, wiedergewinnen und gegebenenfalls erneut als Ausgangsmaterial einsetzen.

Als anorganische Säurechloride kommen bei der Durchführung des erfindungsgemässen Verfahrens alle üblichen anorganischen Säurechloride in Betracht. Vorzugsweise verwendbar sind Thionylchlorid, Phosphortrichlorid oder Phosphorpentachlorid.

Als Katalysatoren können bei der Durchführung des erfindungsgemässen Verfahrens alle diejenigen Verbindungen verwendet werden, die bekanntermassen auch die Umsetzung von Carbonsäuren und ihren Salzen mit anorganischen Säurechloriden zu Carbonsäurechloriden katalysieren (vgl. Houben-Weyl, «Methoden der organischen Chemie», Georg-Thieme-Verlag, Bd. VIII, Seiten 463 ff, Stuttgart 1952, und Bd. E5, Seiten 593 ff (1985)). Vorzugsweise verwendbar sind basische Stickstoffverbindungen. wie tertiäre Amine und Säureamide. Beispielhaft genannt seien Pyridin und Dimethylformamid.

Die Reaktionstemperaturen können bei der Durchführung des erfindungsgemässen Verfahrens innerhalb eines grösseren Bereiches variiert werden. Im allgemeinen arbeitet man bei Temperaturen zwischen 20° C und dem Siedepunkt des Reaktionsgemisches.

Als Verdünnungsmittel können bei der Durchführung des erfindungsgemässen Verfahrens alle üblichen inerten Solventien verwendet werden. Vorzugsweise in Betracht kommen aliphatische und aromatische, gegebenenfalls chlorierte Kohlenwasserstoffe sowie Phosphoroxychlorid und Schwefelkohlenstoff.

Bei der Durchführung des erfindungsgemässen Verfahrens geht man im allgemeinen so vor, dass man die Oxetan-3-carbonsäuren bzw. deren Salze mit einer stöchiometrischen Menge oder auch mit einem Überschuss an anorganischen Säurechloriden sowie gegebenenfalls in Gegenwart eines Katalysators umsetzt. Der stöchiometrische Überschuss an anorganischen Säurechloriden kann dabei 5 bis 300% betragen. Der Katalysator wird im allgemeinen in Mengen von 0,1 bis 20 Gew.-% bezogen auf die eingesetzten Oxetan-3-carbonsäuren bzw. deren Metallsalze zugegeben.

Das Reaktionsgemisch wird nach Massgabe der

Wärme- und/oder Gasentwicklung so lange erhitzt, bis die Umsetzung beendet ist. Zur besseren Reaktionslenkung kann auch in Gegenwart eines inerten Lösungsmittels gearbeitet werden. Im allgemeinen erhitzt man das Reaktionsgemisch bis auf Rückflusstemperatur und behält diese Temperatur bis zur Beendigung der Umsetzung bei. Reaktionsverlauf und Reaktionsende lassen sich nach üblichen Methoden, z. B. durch Gaschromatographie, in einfacher Weise ermitteln.

Die Aufarbeitung des Reaktionsgemisches erfolgt nach üblichen Methoden. Im allgemeinen geht man so vor, dass man das Reaktionsgemisch einer Destillation unterwirft. Nicht umgesetztes anorganisches Säurechlorid wird dabei gesondert aufgefangen und kann zur erneuten Umsetzung verwendet werden. Die Destillationen sind gegebenenfalls im Vakuum und/oder unter Zwischenschaltung einer Kolonne vorzunehmen.

Die nach dem erfindungsgemässen Verfahren herstellbaren 2,2-Bis-chlormethyl-alkancarbonsäurechloride sind wertvolle Zwischenprodukte zur Herstellung anderer Stoffe. So können die 2,2-Bis-chlormethyl-alkancarbonsäurechloride der Formel (I) als Ausgangsmaterialien zur Herstellung von herbizid wirksamen Triazinon-Derivaten bzw. Zur Synthese von fungizid wirksamen Triazolyl-Derivaten verwendet werden.

Es kann z. B. das 2,2-Bis-chlorpivaloylchlorid, gegebenenfalls nach vorherigem Austausch der Chloratome gegen Fluoratome, durch Umsetzung mit Trimethylsilylcyanid in die entsprechenden Halogen-pivaloylcyanide überführt werden, welche sich nach bekannten Methoden zu 1,2,4-Triazin-5-on-Derivaten umsetzen lassen (vgl. DE-OS 3 037 300).

Ferner kann z. B. das 2,2-Bis-chlorpivaloylchlorid durch Behandlung mit Kaliumfluorid in das 2,2-Bis-fluorpivaloylfluorid überführt werden, welches mit Malonsäure-mono-ethylester-Magnesium-Salz zum 2,2-Bis-fluormethylbutan-3-on reagiert. Letzteres lässt sich mit Brom zu dem 2,2-Bis-fluormethyl-4-brom-butan-3-on umsetzen, das nach Reaktion mit 1,2,4-Triazol das 2,2-Bis-fluormethyl-4-(1,2,4-triazol-1-yl)-butan-3-on ergibt. Dieses lässt sich durch Umsetzung mit Cyclohexylmethylbromid und Reduktion des zunächst entstehenden Produktes mit Natriumborhydrid in das 2,2-Bis-fluormethyl-5-cyclohexyl-4-(1,2,4-triazol-1-yl)-pentan-3-ol überführen (vgl. DE-OS 3 326 875, DE-OS 2 951 163 und JP-OS 61 572 (1985)). Die genannten Umsetzungen lassen sich durch das folgende Formelschema wiedergeben:

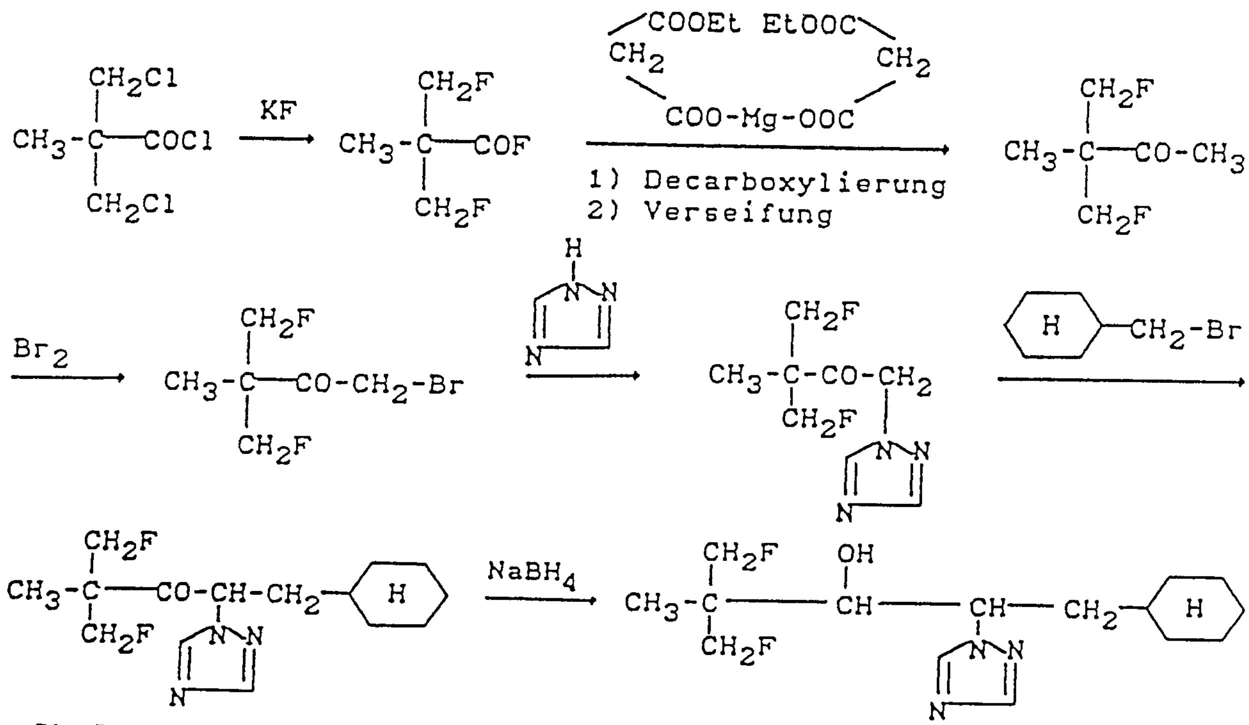


Die Durchführung des erfindungsgemässen Verfahrens wird durch die folgenden Beispiele veranschaulicht.

*Beispiel 1:*

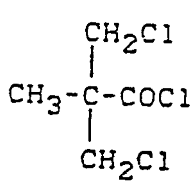

Ein Gemisch aus 11,6 g (0,1 Mol) 3-Methyl-oxetan-3-carbonsäure, 35,7 g (0,3 Mol) Thionylchlorid und 0,2 ml Dimethylformamid wird langsam auf Rückflusstemperatur (etwa 120° C) erhitzt, wobei sich während des Erhitzens Gas entwickelt. Danach wird 5 Stunden bei dieser Temperatur weitergerührt. Die gaschromatographische Untersuchung einer Probe zeigt an, dass sich die eingesetzte 3-Methyl-oxetan-3-carbonsäure vollständig umgesetzt hat. Anschliessend wird überschüssiges Thionylchlorid über eine Destillationsbrücke abdestilliert. Aus dem verbleibenden Rückstand wird bei einem Druck von 10 mbar bei 70-90° C die Hauptfraktion abdestilliert. Man erhält 15,5 g eines Produktes, das gemäss Gaschromatogramm zu 96,1% aus 2,2-Bischlormethyl-propancarbonsäurechlorid besteht.

*Beispiel 2:*

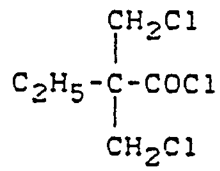

Zu einer Mischung aus 130 g (1 Mol) 3-Ethyloxetan-3-carbonsäure und 1 ml Dimethylformamid werden 476 g (4 Mol) Thionylchlorid getropft. Danach wird so auf Rückflusstemperatur erhitzt, dass ein stetiger Abgasstrom entsteht. Anschliessend wird bei Rückflusstemperatur weitergerührt. Nach insgesamt 32 Stunden wird über eine Brücke destilliert. Im Siedebereich von 95 bis 120° C erhält man bei einem Druck von 10 mbar 166,6 g (0,82 Mol) 2,2-Bis-chlormethyl-butancarbonsäurechlorid, das beim Stehen bei Raumtemperatur erstarrt.

Fp. 38-40° C.

*Herstellung von Ausgangssubstanzen*

*Beispiel 3:*

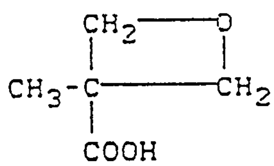

In ein mit Rührer, Innenthermometer und Gaszuleitung versehenes, über einen Heizmantel temperierbares Reaktionsgefäss werden eine Lösung von 20,4 g (0,2 Mol) 3-Methyl-3-hydroxymethyl-oxetan in 100 ml (0,22 Mol) 2,2 m wässeriger Natronlauge, 1 g Aktivkohle mit 5 Gew.-% Palladiumgehalt und 0,030 g $Bi(NO_3)_3 \cdot 5\ H_2O$ eingebracht.

Nach Verdrängen der Luft aus dem Reaktionsgefäss durch Sauerstoff wird der Rührer eingeschaltet und das Reaktionsgemisch auf 80° C erhitzt. Bei dieser Temperatur wird Sauerstoff unter Normaldruck in die Mischung eingeleitet. Nach 3 Stunden sind 0,2 Mol Sauerstoff aufgenommen und die Reaktion kommt zum Stillstand.

Nach dem Abfiltrieren des Katalysators und Nachwaschen mit 20 ml Wasser wird das Filtrat mit 50%iger Schwefelsäure auf pH 1 angesäuert und mit 2 × 50 ml Methylisobutylketon extrahiert. Nach Abziehen des Methylisobutylketons bei 60° C im Vakuum verbleiben als Rückstand 24 g 3-Methyl-oxetan-3-carbonsäure, die gemäss Gaschromatogramm 3 bis 4% an Methylisobutylketon, aber sonst keine weiteren Verunreinigungen enthält. Die Ausbeute errechnet sich danach zu 99% der Theorie. Fp. 58-60° C (nach Umkristallisation aus Ligroin).

*Beispiel 4:*

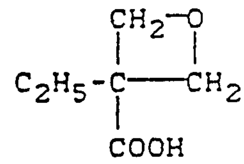

Es wird wie im Beispiel 3 gearbeitet, jedoch mit dem Unterschied, dass 11,6 g (0,1 Mol) 3-Ethyl-3-hydroxymethyl-oxetan in 100 ml 1,2 m wässeriger Natronlauge gelöst und nach Zugabe von 1 g Aktivkohle mit 5 Gew.-% Palladiumgehalt und 30 mg $Bi(NO_3)_3 \cdot 5\ H_2O$ mit Sauerstoff unter Normaldruck bei 80° C oxidiert werden. Nach 90 Minuten ist 0,1 Mol Sauerstoff aufgenommen und die Reaktion kommt zum Stillstand.

Nach dem Abfiltrieren des Katalysators ergibt die Extraktion des mit 50%iger Schwefelsäure auf pH 1 eingestellten Filtrates mit Diethylether 12,9 g eines Produktes, das gemäss Gaschromatogramm zu 99,7% aus 3-Ethyloxetan-3-carbonsäure besteht. Die Ausbeute errechnet sich danach zu 98,9% der Theorie.

Fp. 25° C.

*Beispiel 5:*

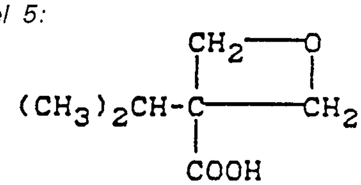

Es wird wie im Beispiel 3 gearbeitet, jedoch mit dem Unterschied, dass 13 g (0,1 Mol) 3-Isopropyl-3-hydroxymethyl-oxetan in 100 ml 1,2 m wässeriger Natronlauge in Gegenwart von 1 g Aktivkohle mit 5 Gew.-% Palladiumgehalt und 30 mg $Bi(NO_3)_3 \cdot 5\ H_2O$ oxidiert werden. Nach 120 Minuten ist 0,1 Mol Sauerstoff aufgenommen, und die Sauerstoffaufnahme ist zum Stillstand gekommen.

Nach dem Abfiltrieren des Katalysators, Ansäuern des Filtrates auf pH 1 und Extraktion mit Diethylether verbleiben nach dem Einengen der organischen Phase unter vermindertem Druck 14,1 g eines Produktes, das zu 94,5% aus 3-Isopropyl-oxetan-3-carbonsäure und zu 3,3% aus 3-Isopropyl-oxetan-3-carbonsäure und zu 3,3% aus 3-Isopropyl-3-hydroxymethyl-oxetan besteht. Die Ausbeute (Selektivität), bezogen auf umgesetztes Ausgangsmaterial, errechnet sich danach zu 96% der Theorie.

Fp. 52-54° C.

**Patentansprüche**

1. Verfahren zur Herstellung von 2,2-Bis-chlormethyl-alkancarbonsäuren der Formel

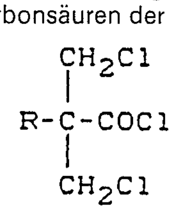

(I)

in welcher
R für Wasserstoff, Alkyl, Cycloalkyl oder gegebenenfalls substituiertes Phenyl steht, dadurch gekennzeichnet, dass man Oxetan-3-carbonsäuren der Formel

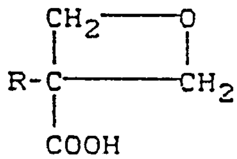

(II)

in welcher
R die oben angegebene Bedeutung hat,
oder deren Salze mit anorganischen Säurechloriden gegebenenfalls in Gegenwart eines Katalysators und gegebenenfalls in Gegenwart eines Verdünnungsmittels bei Temperaturen zwischen 20° C und dem Siedepunkt des Reaktionsgemisches umsetzt.

2. Verfahren gemäss Anspruch 1, dadurch gekennzeichnet, dass man Oxetan-3-carbonsäuren der Formel (II) einsetzt, in denen R für Wasserstoff, Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 4 Kohlenstoffatomen substituiertes Phenyl steht.

3. Verfahren gemäss Anspruch 1, dadurch gekennzeichnet, dass man Oxetan-3-carbonsäuren der Formel (II) in Form von Salzen einsetzt.

4. Verfahren gemäss Anspruch 1, dadurch gekennzeichnet, dass man Thionylchlorid, Phosphortrichlorid oder Phosphorpentachlorid als anorganische Säurechloride einsetzt.

5. Verfahren gemäss Anspruch 1, dadurch gekennzeichnet, dass man basische Stickstoffverbindungen als Katalysatoren einsetzt.

6. Verfahren gemäss Anspruch 5, dadurch gekennzeichnet, dass man tertiäre Amine oder Säureamide als basische Stickstoffverbindungen einsetzt.

7. Verfahren gemäss Anspruch 1, dadurch gekennzeichnet, dass man aliphatische oder aromatische, gegebenenfalls chlorierte Kohlenwasserstoffe, Phosphoroxychlorid oder Schwefelkohlenstoff als Verdünnungsmittel einsetzt.

## Claims

1. Process for the preaparation of 2,2-bischloromethyl-alkanecarboxylic acids of the formula

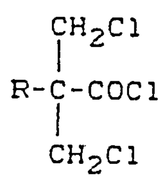

(I)

in which
R represents hydrogen, alkyl, cycloalkyl or optionally substituted phenyl, characterized in that oxetane-3-carboxylic acids of the formula

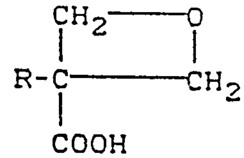

(II)

in which
R has the above-mentioned meaning,
or the salts thereof, are reacted with inorganic acid chlorides, if appropriate in the presence of a catalyst and if appropriate in the presence of a diluent, at temperatures between 20° C and the boiling point of the reaction mixture.

2. Process according to Claim 1, characterized in that oxetane-3-carboxylic acids of the formula (II) are employed in which R represents hydrogen, alkyl having 1 to 12 carbon atoms, cycloalkyl having 3 to 8 carbon atoms or phenyl which is optionally substituted by halogen and/or alkyl having 1 to 4 carbon atoms.

3. Process according to Claim 1, characterized in that oxetane-3-carboxylic acids of the formula (II) in the form of salts are employed.

4. Process according to Claim 1, characterized in that thionyl chloride, phosphorus trichloride or phosphorus pentachloride are employed as inorganic acid chlorides.

5. Process according to Claim 1, characterized in that basic nitrogen compounds are employed as catalysts.

6. Process according to Claim 5, characterized in that tertiary amines or acid amides are employed as basic nitrogen compounds.

7. Process according to Claim 1, characterized in that aliphatic or aromatic, optionally chlorinated hydrocarbons, phosphoroxy chloride or carbon disulphide are employed as diluents.

## Revendications

1. Procédé pour la fabrication d'acides 2,2-bischlorométhylalcanecarboxyliques de formule

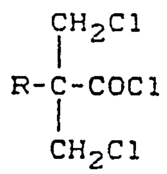

(I)

dans laquelle
R représente l'hydrogène, un goupe alkyle, un groupe cyclo-alkyle ou un groupe phényle éventuellement substitué, caractérisé en ce qu'on fait réagir des acides oxétanne-3-carboxyliques de formule

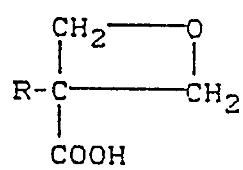

(II)

dans laquelle
R possède la signification décrite ci-dessus, ou

leurs sels, avec des chlorures d'acides inorganiques, éventuellement en présence d'un catalyseur et éventuellement en présence d'un agent de dilution, à des températures comprises entre 20° C et le point d'ébullition du mélange réactionnel.

2. Procédé selon la revendication 1, caractérisé en ce qu'on utilise des acides oxétanne-3-carboxyliques de formule (II) dans laquelle R représente l'hydrogène, un groupe alkyle avec 1 à 12 atomes de carbone, cyclo-alkyle avec 3 à 8 atomes de carbone ou un groupe phényle éventuellement substitué par un halogène et/ou un groupe alkyle avec 1 à 4 atomes de carbone.

3. Procédé selon la revendication 1, caractérisé en ce qu'on utilise les acides oxétanne-3-carboxyliques de formule (II) sous forme de sels.

4. Procédé selon la revendication 1, caractérisé en ce qu'on utilise comme chlorures d'acides inorganiques du chlorure de thionyle, du trichlorure de phosphore ou du pentachlorure de phosphore.

5. Procédé selon la revendication 1, caractérisé en ce qu'on utilise comme catalysateurs des composés basiques d'azote.

6. Procédé selon la revendication 5, caractérisé en ce qu'on utilise comme composés basiques d'azote des amines tertiaires ou des amides d'acides.

7. Procédé selon la revendication 1, caractérisé en ce qu'on utilise comme agents de dilution des hydrocarbures aliphatiques ou aromatiques éventuellement chlorés, l'oxychlorure de phosphore ou le sulfure de carbone.